Europäisches Patentamt

European Patent Office

Office européen des brevets

(19)

(11) Publication number: **0 124 334**
**B1**

(12) **EUROPEAN PATENT SPECIFICATION**

(45) Date of publication of patent specification: **12.07.89**

(21) Application number: **84302744.2**

(22) Date of filing: **24.04.84**

(51) Int. Cl.⁴: **B 01 J 19/00,** C 07 C 5/09,
C 07 C 11/04

(54) Automatic catalyst regeneration and catalyst selectivity estimation.

(30) Priority: **25.04.83 US 488282**

(43) Date of publication of application:
**07.11.84 Bulletin 84/45**

(45) Publication of the grant of the patent:
**12.07.89 Bulletin 89/28**

(84) Designated Contracting States:
**DE FR GB IT**

(56) References cited:
**US-A-3 839 483**
**US-A-3 972 804**
**US-A-4 217 243**
**US-A-4 236 219**
**US-A-4 241 230**

(73) Proprietor: **THE BABCOCK & WILCOX
COMPANY**
**1010 Common Street P.O. Box 60035**
**New Orleans Louisiana 70160 (US)**

(72) Inventor: **Agarwal, Suresh C.**
**5686 Broadview Road Apt. No. 2402**
**Parma Ohio 44134 (US)**

(74) Representative: **Cotter, Ivan John et al**
**D. YOUNG & CO. 10 Staple Inn**
**London WC1V 7RD (GB)**

Courier Press, Leamington Spa, England.

# Description

This invention relates to automatic catalyst regeneration and catalyst selectivity estimation.

In many chemical, petrochemical and synthetic fuel manufacturing processes a raw material feed stream is passed over a catalyst in a reactor either to produce a desired product or to remove certain impurities. The state of the catalyst is of prime importance to these operations because economic operation of a reactor, and consequently the plant, is very much affected by the catalyst state. Product conversion, yield and operating temperature are dependent upon the selectivity of the catalyst.

A catalyst's selectivity decays with time because of a number of factors such as presence of impurities and their concentration in the feed stream, types of byproducts from side reactions, duration of reactor operation, etc. For example, in the selective hydrogenation of acetylene in an ethylene rich stream green oil, polymerisation of green oil on the catalyst surface, etc. occurs. These are deposited on the catalyst surface, thus reducing the surface area available for the reaction. Hence, acetylene hydrogenation to ethylene is reduced. The condition may also lead to a product which is off-specification. Therefore, after operating a reactor with fresh or regenerated catalyst for some time, the catalyst is either regenerated by passing steam through the reactor or replaced by new catalyst, whichever may be appropriate depending on the specific situation.

The state of the art for control of the selective hydrogenation of acetylene into ethylene in the ethylene rich stream is well documented in the following patents:

1. US—A—2 802 889—Frevel et al
2. US—A—2 814 653—Hogan et al
3. US—A—3 113 980—Robinson et al
4. US—A—3 153 679—Rottmayr
5. US—A—3 471 582—Lupfer
6. US—A—3 656 911—Hobbs
7. US—A—3 972 804—McLaughlin et al
8. US—A—4 236 219—Killebrew et al
9. US—A—4 241 230—Drinkard
10. US—A—4 249 907—Callejas

These patents only teach techniques for the temperature control of reactors. They do not provide for any indication of the current state of the catalyst. Consequently, any or all of the following situations may occur:

1. As catalyst selectivity decreases, less and less acetylene will be hydrogenated into ethylene. Thus, the probability of occurrence of an off-specification product increases and ultimately may occur, thus resulting in loss of production.

2. The operator may increase operating temperature of the reactor to compensate for decline in catalyst selectivity on the basis of information from the manufacturer of the catalyst and acetylene concentration in the reactor exit stream. This presents a situation where an operator may be unable to discriminate among the causes of occurrences of more than normal acetylene concentration in the exit stream. This may be a result of several possible conditions, for example, sudden drop in feed temperature, sudden breakthrough of acetylene in cracking furnaces, malfunctioning of intermediate unit operations or their associated components, malfunctioning/failure of instruments, etc.

3. Increase in operating temperature of the reactor results in increasing energy consumption in the feed preheater, thus increasing coolant use in the inter catalyst bed cooler, and increases ethylene hydrogenation to ethane.

Moreover, in the current state of the art in industrial practice, catalyst is regenerated either after occurrence of a severely off-specification product or it has been scheduled by maintenance personnel on the reactor, on the basis of an elapsed period of operation or during plant shutdown. The same is applicable to catalyst change. Therefore, the reactor is normally operated with catalyst for a period longer than the recommended period between two successive regenerations and replacement by fresh catalyst. Therefore, one or more of the aforementioned three conditions may occur or, worse yet, the catalyst may be poisoned. The recommended period of operation between two successive catalyst regenerations is about 18 months. Continued operation without regeneration may poison the catalyst completely, thus reducing its life (normally about 5 years) and may require new catalyst.

As an example of the above-identified patents, US Patent No. US—A—4 241 230 discloses apparatus for use in a hydrogenation process for obtaining a hydrogenated product (such as ethylene) in an exit stream of a reactor from a hydrocarbon raw material (such as acetylene) in a feed stream of the reactor, the apparatus comprising:

first sensor means operatively connected to the feed stream for sensing concentrations of the hydrocarbon raw material and hydrogen in the feed stream;

second sensor means operatively connected to the exit stream for sensing a concentration of the hydrocarbon raw material in the exit stream; and

a control system connected to the first and second sensor means for controlling the processing conditions of the reactor.

However, as stated above, the control effected by the control system of US—A—4 241 230 is restricted to process parameters such as temperature control of the reactor; there is no means of providing any indication of the state of the catalyst.

According to a first aspect of the present invention there is provided apparatus for the automatic regeneration of catalyst used in a selective hydrogenation process for obtaining a hydrogenated product in an exit stream of a reactor, from a hydrocarbon raw material in a feed stream of the reactor, the reactor having regeneration means for regenerating the catalyst, the apparatus comprising:

first sensor means operatively connected to the feed stream for sensing concentrations of the hydrocarbon raw material and hydrogen in the feed stream;

second sensor means operatively connected to the exit stream for sensing a concentration of the hydrocarbon raw material in the exit stream; and

a control system connected to the first and second sensor means and to the regeneration means for calculating a value S for selectivity of the catalyst according to the relationship:

$$S = \frac{C_{H1}}{C_{A1} - C_{A2}},$$

where $C_{H1}$ is the hydrogen concentration in the feed stream, $C_{A1}$ is the hydrocarbon raw material concentration in the feed stream and $C_{A2}$ is the hydrocarbon raw material concentration in the exit stream, and for generating a control signal, when the calculated value for the selectivity approaches a selected value for the selectivity which is indicative that the catalyst should be regenerated, for application to the regeneration means for activating the regeneration means to regenerate the catalyst.

According to a second aspect of the present invention there is provided a method of controlling catalyst regeneration for a selective hydrogenation process of a hydrocarbon raw material in a reactor, the method comprising:

sensing the hydrocarbon raw material concentration in a feed stream of the reactor;

sensing the hydrogen concentration in the feed stream;

sensing the hydrocarbon raw material concentration in an exit stream of the reactor;

calculating an estimated catalyst selectivity according to the relationship

$$S = \frac{C_{H1}}{C_{A1} - C_{A2}},$$

where S = the catalyst selectivity, $C_{A1}$ = the hydrocarbon raw material concentration in the feed stream, $C_{A2}$ = the hydrocarbon raw material concentration in the exit stream, and $C_{H1}$ = the hydrogen concentration in the feed stream; and

regenerating the catalyst of the reactor when the calculated estimated selectivity of the catalyst reaches a selected level.

A preferred embodiment of the present invention described hereinbelow provides a method and apparatus for the automatic regeneration of catalyst in a reactor which is based on the value of the catalyst selectivity. The catalyst is regenerated in a timely fashion, which is important in view of the high cost of catalyst and the possibility of poisoning the catalyst beyond the point where it can be regenerated. The preferred method and apparatus are applicable to the automatic regeneration of catalyst in a reactor wherein selective hydrogenation of specific unsaturated hydrocarbon is conducted for its removal from an olefin rich stream. A standby reactor is available in the preferred apparatus for the automatic regeneration of catalyst in a primary reactor. The apparatus can operate regardless of the type of reactor or catalyst except for the fluidised bed type of reactor.

The apparatus according to the preferred embodiment may include an auxiliary reactor having fresh or regenerated catalyst used in the process, the auxiliary reactor being connected to the feed and exit streams over valves which are connected to and controlled by the control system to transfer the feed stream to the auxiliary reactor when the first mentioned reactor is undergoing regeneration of its catalyst.

The process may be the selective hydrogenation of a specific unsaturated hydrocarbon forming the raw material, for its removal from an olefin rich stream, the first and second sensor means being operable to sense a concentration of the hydrocarbon in the feed and exit streams respectively with the first sensor means also being operable to sense a concentration of hydrogen in the feed stream, and the control system including calculator means for calculating the selectivity of the catalyst according to the relationship

$$C_{H1}/(C_{A1} - C_{A2}).$$

The invention may be embodied so as to provide a method of regenerating catalyst in an automatic manner using a Network 90 system available from the Bailey Meter Company.

The preferred apparatus for automatically regenerating catalyst, described hereinbelow, is simple in design, rugged in construction and economical to manufacture.

The invention will now be further described, by way of illustrative and non-limiting example, with reference to the accompanying drawings, in which:

Figure 1 is a schematic representation of an automatic catalyst regeneration apparatus or system embodying the invention showing dual reactors with control and sensing elements;

Figure 2 is a block diagram showing additional details of a control computer system used in the apparatus;

Figure 3 is a block diagram showing additional details of a unit employed in the control computer system for establishing an actual catalyst selectivity value;

Figure 4 is a block diagram showing additional details of a unit in the control computer system for determining whether the actual selectivity calculated has reached a selected limit for the selectivity;

Figure 5 is a block diagram showing a series of three sub-units in the control computer system which control the operation of the overall apparatus to transfer operation from one of the reactors to the other reactor and thereafter regenerate the catalyst of the first reactor; and

Figure 6 is a block diagram showing additional details of units in the control computer system for monitoring catalyst status and for setting various time durations used in controlling the apparatus using the control elements of Figure 5.

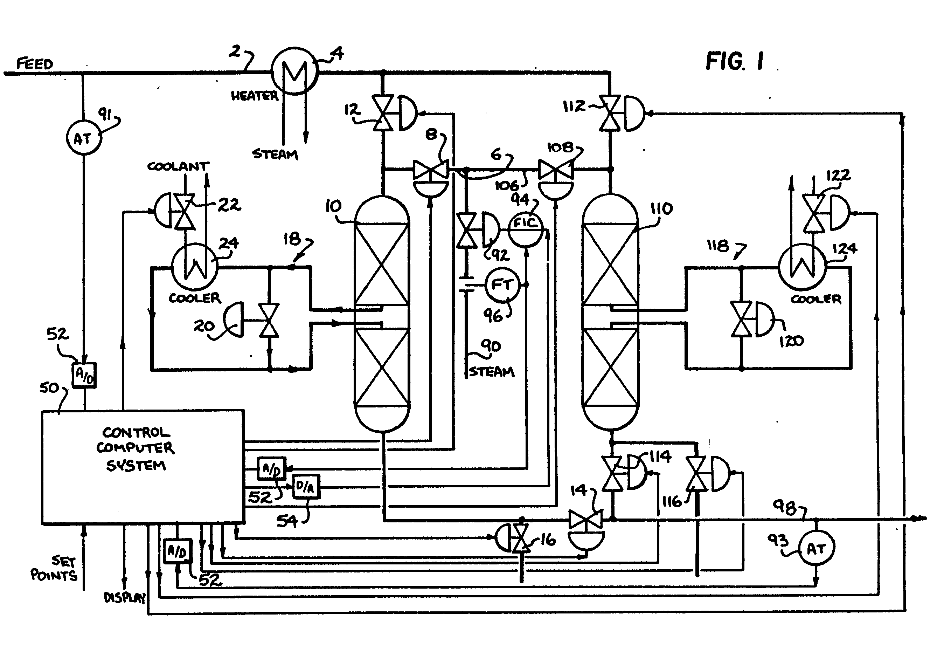

Referring to the drawings, Figure 1 shows an apparatus for automatically regenerating a catalyst in one of two reactors 10 and 110 which are of substantially identical design.

The reactor 10 is used as a primary reactor in a process for obtaining a product from a raw material using a catalyst, with the other reactor 110 being utilised as an auxiliary reactor during the period when the catalyst in the primary reactor is being regenerated.

The specific example illustrated involves a process for the selective hydrogenation of acetylene to ethylene. The invention is suitable, however, in the regeneration of catalysts in other processes.

As shown in Figure 1, a feed stream containing ethylene, acetylene and hydrogen is provided over a feed line 2, past a heater 4 which, for example, uses steam and to an inlet valve 12 at an inlet of the primary reactor 10. Since the reactors 10 and 110 are substantially identical, corresponding parts in the two reactor systems are designated by the same reference numerals, except that the numerals associated with the auxiliary reactor 110 are increased by 100 with respect to those associated with the reactor 10.

A steam line 6 is connected to the inlet of the reactor 10 and includes a regenerator valve 8. The line 6 is connected to a steam source 90 via a main steam valve 92 controlled by a FIC controller 94 which reacts to a computer control signal as well as a flow transmitter 96. With the valves 8 and 92 open, steam is provided to catalyst in the reactor 10 to regenerate the catalyst.

In normal operation of the reactor 10 for producing ethylene, however, at least the valve 8 is closed. A line connects an outlet of reactor 10 to an outlet valve 14. A drain valve 16 is connected to a drain line upstream of the outlet valve 14 for draining waste products of the regeneration cycle.

An exit line 98 is connected to the outlet valve 14 of the reactor 10 as well as to the outlet valve 114 of the reactor 110.

Each of the reactors 10, 110 comprises two reactor beds separated by an intermediate cooling bed. The cooling bed of the reactor 10 is connected to a cooling circuit 18 having a bypass valve 20 for regulating the temperature in the reactor 10. Coolant is supplied to a heat exchanger 24 over a coolant valve 22. An identical cooling circuit 118 is provided for the auxiliary reactor 110.

A chromatograph 91 measures the concentration of acetylene and hydrogen in the feed stream of the feed line 2 and a second chromatograph 93 measures the concentration of acetylene in the exit stream of the exit line 98. The chromatographs 91 and 93 may be replaced by any other suitable sensor(s) for sensing raw material concentration in the feed and exit lines.

All sensors are connected to a control computer system 50 via analog to digital converters 52. The steam valve controller 94 is connected over a digital to analog converter 54 to the control computer system 50.

Instrumentation and control schemes for the reactors 10 and 110 are utilised for modulating the systems, as well as for start-up and shutdown operations. Specific details of the operation of the separate systems are known in the art and demonstrated in the above-identified US Patents.

The primary reactor 10 is initially used for the selective hydrogenation process while the reactor 110 contains regenerated or fresh catalyst that is available for subsequent use. In this state of the apparatus, the valves 12, 22 and 14 are open while the valves 8 and 16 are closed. All of the corresponding valves (108, 112, 114, 116, and 122) in the auxiliary reactor system are closed. The valve 20 is active for temperature control purposes in the selective hydrogenation process, while the valve 120 is inactive. The valve 92 is closed since it is used for modulating control during the regeneration process.

As will become apparent hereinbelow, the key features of the present control structure and method are:

1. estimation of catalyst selectivity;
2. detection of catalyst selectivity below a normal required level;
3. transfer of selective hydrogenation to the auxiliary reactor 110 upon detection of an unsatisfactory condition in the catalyst of the primary reactor 10;
4. subsequent regeneration of catalyst in the reactor 10;
5. bringing the primary reactor 10 to a ready state for the takeover of selective hydrogenation from the reactor 110; and
6. catalyst status accounting.

A measurement of catalyst selectivity towards acetylene hydrogenation to ethylene is obtained by the algorithm:

$$S = \frac{C_{H1}}{C_{A1} - C_{A2}},$$

where
S = selectivity of the catalyst;
$C_{A1}$ = acetylene concentration in the feed stream;
$C_{A2}$ = acetylene concentration in the exit stream; and
$C_{H1}$ = hydrogen concentration in the feed stream.

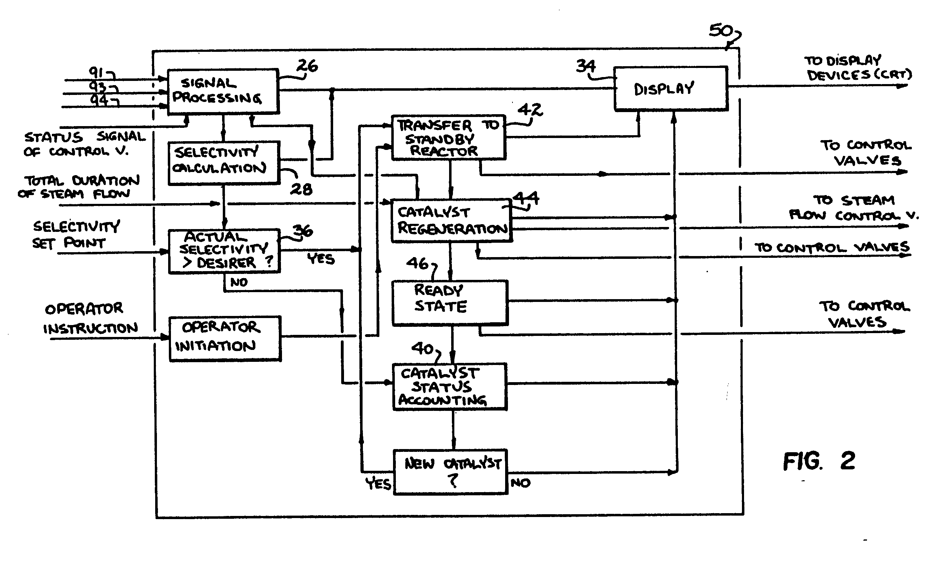

Acetylene and hydrogen concentration in the feed stream are provided by the chromatograph 91. Acetylene concentration in the exit stream is provided by the chromatograph 93. Signals representing these concentrations are provided to the control computer system 50 where they are processed for noise and converted to engineering units in a signal processing block 26 shown in Figure 2. This is based on known principles of signal processing.

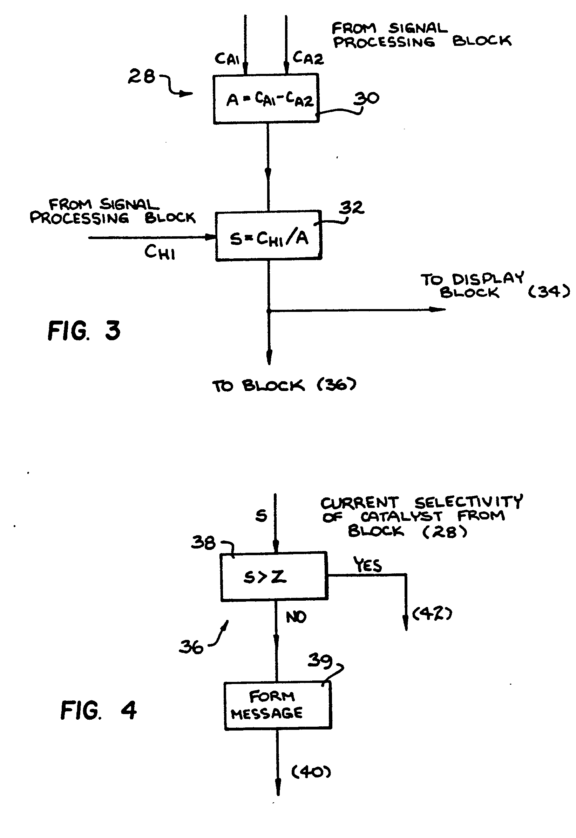

The signals are then provided to a selectivity calculation block 28 where the selectivity calculation is performed according to the algorithm set forth above. Details of the block 28 are shown in Figure 3, where a subtraction unit 30 takes the value of the difference between the feed and exit stream acetylene concentrations which value forms the divisor of the hydrogen concentration in a division unit 32. The output of the unit 32 is sent to a display block 34 as well as to a desired selectivity block 36. The current selectivity value for the catalyst is thus displayed to the operator by the display block 34, which may be of known design.

Details of the block 36, for determining whether the catalyst selectivity has fallen to a set limit value Z, are shown in Figure 4. The value Z is set in a limit unit 38 and is based on manufacturer's recommendation and operating experience with the particular catalyst being used.

If the actual selectivity is still above the limit Z, a signal is supplied over a form message unit 39 to a catalyst status accounting block 40. When actual selectivity has fallen to the limit, a signal is sent to a transfer block 42 for initiating transfer of the process from the primary reactor 10 to the auxiliary reactor 110.

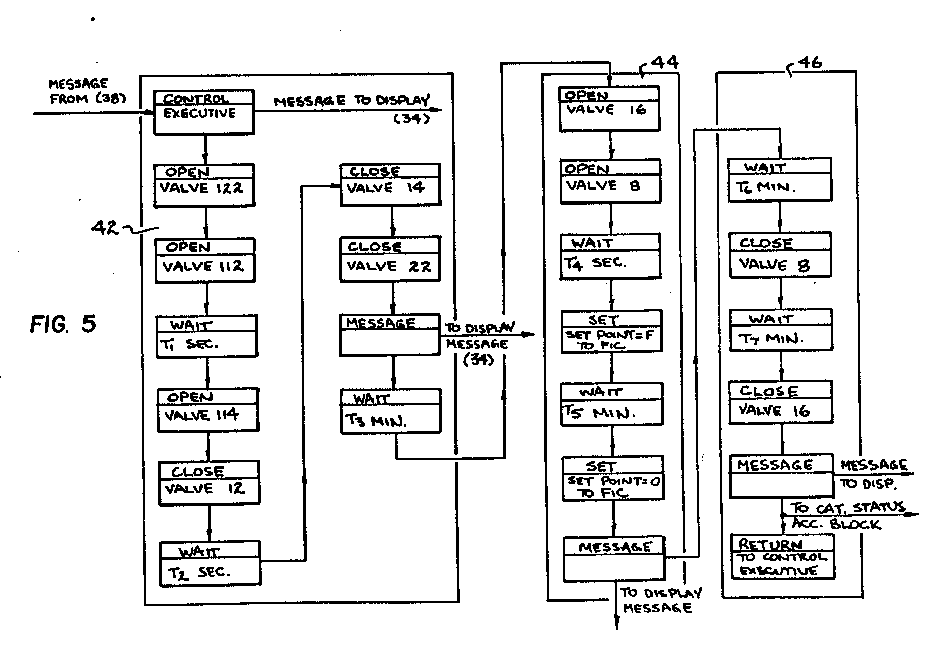

Figure 5 shows details of the transfer block 42 as well as a catalyst regeneration block 44 and a ready state block 46.

As shown in Figure 5, the valves in the apparatus of Figure 1 are controlled in appropriate sequence and with time delays where necessary.

The valve 122 is first opened to supply coolant to the heat exchanger 124 in the cooling circuit 118 of the auxiliary reactor 110. The valve 112 is then opened to establish a flow of raw materials from the feed line 2 to the reactor 110. A waiting period of T1 seconds is then observed to fill the reactor 110 to an appropriate level, after which the outlet valve 114 is opened for establishing a flow of products to the exit line 98. The inlet valve 12 of the reactor 10 is then closed and a second waiting period of T2 seconds is observed. After this time, which permits drainage of the primary reactor 10, the outlet valve 14 is closed. After this the coolant valve 22 is closed and a message is provided to the display 34 for the purpose of informing the operator that the initial transfer steps have been taken.

A waiting period of T3 minutes is then observed, after which regeneration for the primary reactor 10 is commenced. To achieve these control functions, the block 44 first opens the drain valve 16. The regeneration valve 8 is then opened to commence the regeneration of catalyst in the reactor 10. The remainder of the steps illustrated in the block 44 of Figure 5 complete the regeneration process in known fashion using the controller 94 and flow transmitter 96 further in conjunction with appropriate programming in the control computer system 50.

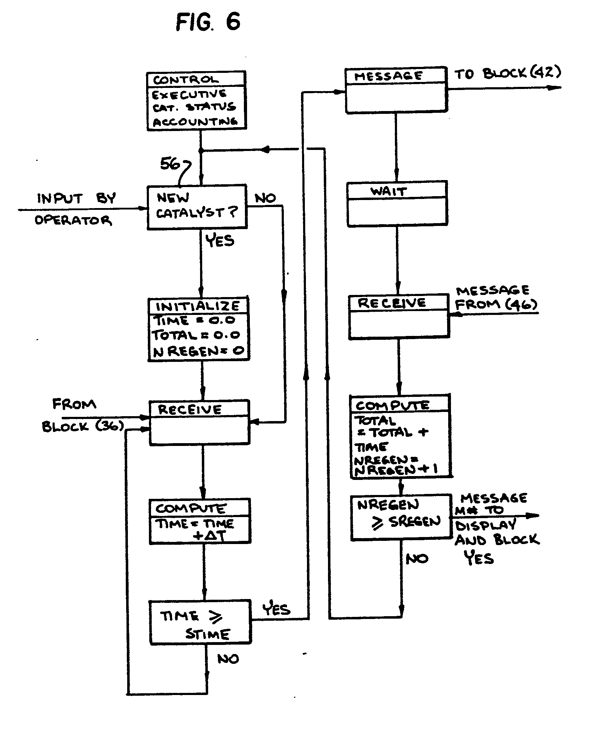

When regeneration is complete, a message signal is again provided to the display 34 for operator information. The regeneration valve 8 is then closed by appropriate controls in the ready state block 46. After observing an appropriate waiting period T7 for drainage of the reactor 10, the drain valve 16 is closed and a further message is sent to the display 34 which indicates that the primary reactor 10 is now again available for further use. A last step in the automatic regeneration system is an accounting for the total time between two successive regenerations, the total time for use of catalyst and the total number of regenerations. This is accomplished in the control block 40 which is shown in greater detail in Figure 6.

The circuit of Figure 6 permits the operator to initiate manually a regeneration step over a block 56. Regeneration also is begun whenever the total time between successive regenerations is greater than a specified time TOTALT. Regeneration may also be initiated when the total time for usage exceeds a total specified time STIME. The number of regenerations NGEGEN is also counted in the circuitry of Figure 6 with a new catalyst being provided after a selected number of regeneration cycles SREGEN.

The various messages which are provided to the operator by the various blocks are as follows:

1. reactor 10 to be switched to reactor 110 automatically;
2. reactor switchover complete;
3. catalyst regeneration process started;
4. catalyst being regenerated in reactor;
5. catalyst regeneration complete;
6. reactor being switched because of
    (a) selectivity below acceptable level, or
    (b) operator demand, or
    (c) operating duration at selected limit; and
7. new catalyst required.

**Claims**

1. Apparatus for the automatic regeneration of catalyst used in a selective hydrogenation process for obtaining a hydrogenated product in an exit stream of a reactor (10), from a hydrocarbon raw material in a feed stream of the reactor, the reactor having regeneration means (92, 94, 96) for regenerating the catalyst, the apparatus comprising:

   first sensor means (91) operatively connected to the feed stream for sensing concentrations of the hydrocarbon raw material and hydrogen in the feed stream;

   second sensor means (93) operatively connected to the exit stream for sensing a concentration of the hydrocarbon raw material in the exit stream; and

   a control system (50) connected to the first and second sensor means (91, 93) and to the regeneration means (92, 94, 96) for calculating a value S for selectivity of the catalyst according to the relationship:

$$S=\frac{C_{H1}}{C_{A1}-C_{A2}},$$

where $C_{H1}$ is the hydrogen concentration in the feed stream, $C_{A1}$ is the hydrocarbon raw material concentration in the feed stream and $C_{A2}$ is the hydrocarbon raw material concentration in the exit stream, and for generating a control signal, when the calculated value (S) for the selectivity approaches a selected value (Z) for the selectivity which is indicative that the catalyst should be regenerated, for application to the regeneration means for activating the regeneration means to regenerate the catalyst.

2. Apparatus according to claim 1, including an auxiliary reactor (110) containing catalyst for the process and connected to the exit stream and the feed stream, and valve means operatively engaged in at least one of the exit and feed streams and connected to the control system (50) for supplying hydrocarbon raw material via the exit stream to the auxiliary reactor (110) when the regeneration means (92, 94, 96) are activated to regenerate catalyst in the first-mentioned reactor (10).

3. Apparatus according to claim 1 or claim 2, wherein the control system (50) includes calculation means (28) for calculating the catalyst selectivity value (S).

4. Apparatus according to claim 3, including desired selectivity means (36) for receiving the selected selectivity value (Z) and connected to the calculation means (28), the desired selectivity means (36) being operable to generate the control signal when the calculated selectivity value (S) approaches the selected selectivity value (Z).

5. Apparatus according to claim 4, including catalyst status accounting means (40) connected to the desired selectivity means (36) for receiving a signal from the desired selectivity means when the calculated selectivity value (S) has not approached the selected selectivity value (Z), the catalyst status accounting means (40) being operable to determine a time period during which the process is conducted with the catalyst before it is regenerated.

6. Apparatus according to claim 5, wherein the catalyst status accounting means (40) is connected to the catalyst regeneration means (92, 94, 96), the catalyst status accounting means (40) having desired time period setting means operable to compare a desired time period for use of the catalyst with the actual time period during which the catalyst has been used and, when the actual time period reaches the desired time period, activating the regeneration means.

7. Apparatus according to claim 5 or claim 6, wherein the catalyst status accounting means (40) includes regeneration counting means for counting the number of times the catalyst in the reactor has been regenerated, and display means (34) is connected to the catalyst status accounting means (40) for displaying a message when the number of times the catalyst has been regenerated reaches a selected number of times for catalyst regeneration.

8. Apparatus according to claim 7 as ultimately dependent on claim 2, wherein the display means (34) is operable to display signals indicative of a transfer of the process from one reactor to the other reactor, the beginning of catalyst regeneration by the regeneration means (92, 94, 96), a ready state of the reactor for which catalyst is being regenerated, and the number of times the catalyst has been regenerated, and wherein the accounting means (40) is operable to store the time that catalyst is utilised in the process of said one reactor, the number of times catalyst is regenerated in said one reactor and a maximum desired number of regeneration cycles and time duration for catalyst use.

9. A method of controlling catalyst regeneration for a selective hydrogenation process of a hydrocarbon raw material in a reactor (10), the method comprising:

sensing (91) the hydrocarbon raw material concentration in a feed stream of the reactor (10);

sensing (91) the hydrogen concentration in the feed stream;

sensing (93) the hydrocarbon raw material concentration in an exit stream of the reactor;

calculating an estimated catalyst selectivity according to the relationship

$$S=\frac{C_{H1}}{C_{A1}-C_{A2}},$$

where S=the catalyst selectivity, $C_{A1}$=the hydrocarbon raw material concentration in the feed stream, $C_{A2}$=the hydrocarbon raw material concentration in the exit stream, and $C_{H1}$=the hydrogen concentration in the feed stream; and

regenerating the catalyst of the reactor (10) when the calculated estimated selectivity (S) of the catalyst reaches a selected level (Z).

10. A method according to claim 9, including displaying (34) the calculated catalyst selectivity (S) to an operator of the reactor.

11. A method according to claim 9 or claim 10, including measuring the total time during which the catalyst is used in the process and regenerating the catalyst after the total time has reached a selected time duration for use of the catalyst.

12. A method according to claim 9, claim 10 or claim 11, including transferring feed of the feed stream to an auxiliary reactor (110) when the first-mentioned reactor (10) is being regenerated, the auxiliary reactor having one of regenerated and fresh catalyst.

13. A method according to any one of claims 9 to 12, wherein the hydrocarbon raw material is acetylene.

**Patentansprüche**

1. Vorrichtung zur automatischen Regenierung von Katalysator, der in einem selektiven Hydrierverfahren zur Gewinnung eines hydrierten Produktes in einem Ausgangsstrom eines Reaktors (10) aus einem Kohlenwasserstoffrohmaterial in einem Beschickungsstrom des Reaktors verwendet wird, wobei der Reaktor Regeneriereinrichtungen (92, 94, 96) zur Regenerierung des Katalysators hat, mit

einer ersten Meßwertgebereinrichtung (91), die funktionsmäßig mit dem Beschickungsstrom verbunden ist, um Konzentrationen des Kohlenwasserstoffrohmaterials und von Wasserstoff in dem Beschickungsstrom abzufühlen,

einer zweiten Meßwertgebereinrichtung (93), die funktionsmäßig mit dem Ausgangsstrom verbunden ist, um eine Konzentration des Kohlenwasserstoffrohmaterials in dem Ausgangsstrom abzufühlen, und

einem Steuersystem (50), das mit der ersten und zweiten Meßwertgebereinrichtung (91, 93) und mit den Regeneriereinrichtungen (92, 94, 96) verbunden ist, um einen Wert S für die Selektivität des Katalysators nach der Gleichung

$$S = \frac{C_{H1}}{C_{A1} - C_{A2}}$$

zu berechnen, worin $C_{H1}$ die Wasserstoffkonzentration des Beschickungsstromes ist, $C_{A1}$ des Kohlenwasserstoffrohmaterialkonzentration in dem Beschickungsstrom ist und $C_{A2}$ die Kohlenwasserstoffrohmaterialkonzentration in dem Ausgangsstrom ist, und um ein Kontrollsignal zu erzeugen, wenn der berechnete Wert (S) für die Selektivität sich einem ausgewählten Wert (Z) für die Selektivität nähert, welcher ein Anzeichen dafür ist, daß der Katalysator regeneriert werden sollte, und es an die Regeneriereinrichtungen zur Aktivierung der Regeneriereinrichtungen, um den Katalysator zu regenerieren, zu geben.

2. Vorrichtung nach Anspruch 1 mit einem Hilfsreaktor (110), der Katalysator für das Verfahren enthält und mit dem Ausgangsstrom und dem Beschickungsstrom verbunden ist, und einer Ventileinrichtung, die funktionsmäßig in wenigstens einen der Ausgangs- und Beschickungsströme eingreift und mit dem Steuersystem (50) verbunden ist, um Kohlenwasserstoffrohmaterial über den Ausgangsstrom zu dem Hilfsreaktor (110) zu führen, wenn die Regeneriereinrichtungen (92, 94, 96) aktiviert werden, um Katalysator in dem ersterwähnten Reaktor (10) zu regenerieren.

3. Vorrichtung nach Anspruch 1 oder Anspruch 2, worin das Steuersystem (50) Rechnereinrichtungen (28) zur Errechnung des Katalysatorselektivitätswertes (S) enthält.

4. Vorrichtung nach Anspruch 3 mit erwünschten Selektivitätseinrichtungen (36) zum Empfang des ausgewählten Selektivitätswertes (Z), die mit der Rechnereinrichtung (28) verbunden sind, wobei die erwünschten Selektivitätseinrichtungen (36) so arbeiten können, daß sie das Kontrollsignal erzeugen, wenn der errechnete Selektivitätswert (S) sich dem ausgewählten Selektivitätswert (Z) nähert.

5. Vorrichtung nach Anspruch 4 mit einer den Katalysatorzustand errechnenden Einrichtung (40), die mit der erwünschten Selektivitätseinrichtung (36) verbunden ist, um ein Signal von der erwünschten Selektivitätseinrichtung aufzunehmen, wenn der berechnete Selektivitätswert (S) sich nicht dem ausgewählten Selektivitätswert (Z) genähert hat, wobei die Katalysatorzustandberechnungseinrichtung (40) so betrieben werden kann, daß sie eine Zeitperiode bestimmen kann, während welcher das Verfahren mit dem Katalysator durchgeführt wird, bevor er regeneriert wird.

6. Vorrichtung nach Anspruch 5, bei der die Katalysatorzustandberechnungseinrichtung (40) mit den Katalysatorregeneriereinrichtungen (92, 94, 96) verbunden ist, wobei die Katalysatorzustandberechnungseinrichtung (40) Einrichtungen zur Einstellung der erwünschten Zeitperiode hat, die so arbeiten können, daß sie eine erwünschte Zeitperiode für die Verwendung des Katalysators mit der tatsächlichen Zeitperiode vergleichen, während welcher der Katalysator verwendet wurde, und die Regeneriereinrichtungen aktiviert, wenn die tatsächliche Zeitperiode die erwünschte Zeitperiode erreicht.

7. Vorrichtung nach Anspruch 5 oder Anspruch 6, bei der die Katalysatorzustandberechnungseinrichtung (40) Regenerierungszähleinrichtungen für das Zählen der Anzahl, in der der Katalysator im Reaktor regeneriert wurde, enthält, und daß eine Anzeigeeinrichtung (34) mit der Katalysatorzustandberechnungseinrichtung (40) verbunden ist, um eine Mitteilung zu machen, wenn die Anzahl, in welcher der Katalysator regeneriert wurde, eine ausgewählte Anzahl für Katalysatorregenerierungen erreicht.

8. Vorrichtung nach Anspruch 7 und letztlich in Abhängigkeit von Anspruch 2, bei der die Anzeigeeinrichtung (34) so betrieben werden kann, daß sie Signale anzeigt, die ein Anzeichen für eine Überführung des Verfahrens von einem Reaktor zu dem anderen Reaktor, für den Beginn der Katalysatorregenerierung durch die Regeneriereinrichtungen (92, 94, 96), einen fertigen Zustand des Reaktors, für welchen Katalysator regeneriert wird, und die Anzahl, in der der Katalysator regeneriert wurde, sind und worin die Zähleinrichtung (40) so betrieben werden kann, daß sie die Zeit, in der Katalysator in dem Verfahren des einen Reaktors benutzt wird, die Anzahl, in der Katalysator in dem einen Reaktor regeneriert wird, und eine maximal erwünschte Anzahl von Regenerierungszyklen und die Zeitdauer für die Katalysatorverwendung speichert.

9. Verfahren zur Steuerung der Katalysatorregenerierung für ein selektives Hydrierverfahren eines Kohlenwasserstoffrohmaterials in einem Reaktor (10), wobei das Verfahren darin besteht, daß man

die Kohlenwasserstoffrohmaterialkonzentration in einem Beschickungsstrom des Reaktors (10) anfühlt (91),

die Wasserstoffkonzentration in dem Beschickungsstrom abfühlt (91),

die Kohlenwasserstoffrohmaterialkonzentra-

tion in einem Ausgangsstrom des Reaktors abführt (93),

eine geschätzte Katalysatorselektivität nach der Gleichung

$$S = \frac{C_{H1}}{C_{A1} - C_{A2}},$$

worin S = die Katalysatorselektivität, $C_{A1}$ = die Kohlenwasserstoffrohmaterialkonzentration in dem Beschickungsstrom, $C_{A2}$ = die Kohlenwasserstoffrohmaterialkonzentration in dem Ausgangsstrom und $C_{H1}$ = die Wasserstoffkonzentration in dem Beschickungsstrom, berechnet und

den Katalysator des Reaktors (10) regeneriert, wenn die berechnete geschätzte Selektivität (S) des Katalysators einen ausgewählten Wert (Z) erreicht.

10. Verfahren nach Anspruch 9, bei dem man die berechnete Katalysatorselektivität (S) einem Betreiber des Reaktors anzeigt (34).

11. Verfahren nach Anspruch 9 oder Anspruch 10, bei dem man die Gesamtzeit mißt, während welcher der Katalysator in dem Verfahren verwendet wird, und den Katalysator regeneriert, nachdem die Gesamtzeit eine ausgewählte Zeitdauer für die Verwendung des Katalysators erreicht hat.

12. Verfahren nach Anspruch 9, Anspruch 10 oder Anspruch 11, bei dem man Beschickung des Beschickungsstromes zu einem Hilfsreaktor (110) überführt, wenn der ersterwähnte Reaktor (10) regeneriert wird, wobei der Hilfsreaktor regenerierten und frischen Katalysator besitzt.

13. Verfahren nach einem der Ansprüche 9 bis 12, bei dem das Kohlenwasserstoffrohmaterial Acetylen ist.

## Revendications

1. Appareillage pour la régénération automatique d'un catalyseur utilisé lors d'un procédé d'hydrogénation sélective destiné à donner un produit hydrogéné dans un courant de sortie d'un réacteur (10), à partir d'une matière première hydrocarbonée dans un courant de charge du réacteur, le réacteur possédant des moyens de régénération (92, 94, 96) pour régénérer le catalyseur, l'appareillage comprenant:

un premier moyen capteur (91), raccordé d'une manière opérationnelle au courant de charge, destiné à capter les concentrations de la matière première hydrocarbonée et de l'hydrogène dans le courant de charge;

un deuxième moyen capteur (93), raccordé d'une manière opérationnelle au courant de sortie, destiné à capter la concentration de la matière première hydrocarbonée dans le courant de sortie; et

un système de régulation (50), raccordé au premier et au deuxième moyens capteurs (91, 93) et aux moyens de régénération (92, 94, 96), destiné à calculer une valeur S de la sélectivité du catalyseur selon la relation suivante:

$$S = C_{H1}/(C_{A1} - C_{A2})$$

où $C_{H1}$ est la concentration de l'hydrogène dans le courant de charge, $C_{A1}$ est la concentration de la matière première hydrocarbonée dans le courant de charge et $C_{A2}$ est la concentration de la matière première hydrocarbonée dans le courant de sortie, et aussi à produire un signal de régulation quand la valeur calculée (S) pour la sélectivité se rapproche d'une valeur sélectionnée (Z) de la sélectivité, cette dernière valeur indiquant que le catalyseur devrait être régénéré, pour application au moyen de régénération, dans le but d'activer le moyen de régénération et ainsi régénérer le catalyseur.

2. Appareillage selon la revendication 1, comprenant un réacteur auxiliaire (110) contenant un catalyseur du procédé et raccordé au courant de sortie et au courant de charge, et un moyen de vannes, installé d'une manière opérationnelle dans au moins le courant de sortie ou le courant de charge et raccordé au système de régulation (50) pour envoyer, par l'intermédiaire du courant de sortie, de la matière première hydrocarbonée au réacteur auxiliaire (110) quand les moyens de régénération (92, 94, 96) sont activés pour régénérer le catalyseur dans le réacteur (10) mentionné en premier.

3. Appareillage selon la revendication 1 ou la revendication 2, dans lequel le système de régulation (50) comprend un moyen de calcul (28) destiné à calculer la valeur (S) de la sélectivité du catalyseur.

4. Appareillage selon la revendication 3, comprenant un moyen de sélectivité de consigne (36) destiné à recevoir la valeur (Z) de la sélectivité sélectionnée et raccordé au moyen de calcul (28), le moyen de sélectivité de consigne (36) pouvant être exploité de façon à produire le signal de régulation quand la valeur calculée (S) de la sélectivité s'approche de la valeur sélectionnée (Z) de la sélectivité.

5. Appareillage selon la revendication 4, comprenant un moyen (40) de détermination de l'état du catalyseur, raccordé au moyen de sélectivité de consigne (36) pour recevoir un signal du moyen de sélectivité de consigne quand la valeur calculée (S) de la sélectivité ne s'est pas approchée de la valeur sélectionnée (Z) de la sélectivité, le moyen de détermination (40) de l'état du catalyseur pouvant être exploité de façon à déterminer une période de temps pendant laquelle le procédé est mis en oeuvre avec le catalyseur avant sa régénération.

6. Appareillage selon la revendication 5, dans lequel le moyen (40) de détermination de l'état du catalyseur est raccordé aux moyens de régénération du catalyseur (92, 94, 96), le moyen (40) de détermination de l'état du catalyseur comportant un moyen de réglage de la période de temps souhaitée, pouvant être utilisé pour comparer une période de temps souhaitée pour utilisation du catalyseur à la période de temps effective au cours de laquelle le catalyseur a été utilisé et, quand la période de temps effective atteint la période de temps souhaitée, activant le moyen de régénération.

7. Appareillage selon la revendication 5 ou la revendication 6, dans lequel le moyen (40) de détermination de l'état du catalyseur comprend un moyen de comptage des régénérations, destiné à compter le nombre de fois que le catalyseur dans le réacteur a été régénéré, et le moyen d'affichage (34) est raccordé au moyen (40) de détermination de l'état du catalyseur pour afficher un message quand le nombre de fois que le catalyseur a été régénéré atteint un nombre de fois sélectionné pour la régénération du catalyseur.

8. Appareillage selon la revendication 7, dans la mesure où, en fin de compte, elle dépend de la revendication 2, dans lequel le moyen d'affichage (34) peut être utilisé pour afficher des signaux indiquant un transfert du procédé d'un réacteur à l'autre réacteur, le début de la régénération du catalyseur par le moyen de régénération (92, 94, 96), un état du réacteur "prêt", état dans lequel le catalyseur est en cours de régénération, et le nombre de fois que le catalyseur a été régénéré, et dans lequel le moyen de détermination (40) peut être utilisé pour mémoriser le temps utilisé par le catalyseur dans le procédé dudit un réacteur, le nombre de fois que le catalyseur est régénéré dans ledit un réacteur, et un nombre maximal de consigne de cycles de régénération, ainsi que la durée d'utilisation du catalyseur.

9. Procédé de régulation de la régénération du catalyseur dans un procédé d'hydrogénation sélective d'une matière première hydrocarbonée dans un réacteur (10) procédé consistant

à capter (91) la concentration de la matière première hydrocarbonée dans un courant de charge du réacteur (10);

à capter (91) la concentration de l'hydrogène dans le courant de charge;

à capter (93) la concentration de la matière première hydrocarbonée dans un courant de sortie du réacteur;

à calculer une sélectivité estimée du catalyseur selon la relation

$$S=C_{H1}/(C_{A1}-C_{A2})$$

où S est la sélectivité du catalyseur, $C_{A1}$ la concentration de la matière première hydrocarbonée dans le courant de charge, $C_{A2}$ la concentration de la matière première hydrocarbonée dans le courant de sortie, et $C_{H1}$ la concentration de l'hydrogène dans le courant de charge; et

à régénérer le catalyseur du réacteur (10) quand la sélectivité estimée calculée (S) du catalyseur atteint une valeur sélectionnée (Z).

10. Procédé selon la revendication 9, consistant à afficher (34) la sélectivité calculée (S) du catalyseur, à l'attention d'un opérateur du réacteur.

11. Procédé selon la revendication 9 ou la revendication 10, comprenant l'étape consistant à mesurer le temps total pendant lequel le catalyseur est utilisé dans le procédé, et à régénérer le catalyseur après que le temps total a atteint une durée de temps sélectionnée d'utilisation du catalyseur.

12. Procédé selon la revendication 9, la revendication 10 ou la revendication 11, comprenant l'étape consistant à transférer la charge du courant de charge vers un réacteur auxiliaire (110) quand le réacteur (10) mentionné en premier est en cours de régénération, le réacteur auxiliaire étant un réacteur à catalyseur régénéré et frais.

13. Procédé selon l'une quelconque des revendications 9 à 12, dans lequel la matière première hydrocarbonée est l'acétylène.

FIG. I

FEED

2
HEATER
M
4
STEAM

91
AT

COOLANT
22
24
COOLER
18
20

52
A/D
50

10

12
8
6
106
108
92
94
FIC
FT
96
90
STEAM

112

122
124
COOLER
118
120
110

CONTROL
COMPUTER
SYSTEM

A/D
52
D/A
54

14
16
114
116

98
93
AT

SET
POINTS

DISPLAY

A/D
52

EP 0 124 334 B1

FIG. 2

EP 0 124 334 B1

28 →  CA1 ↓  CA2 ↓  FROM SIGNAL PROCESSING BLOCK

$$A = C_{A1} - C_{A2}$$  30

FROM SIGNAL PROCESSING BLOCK

$C_{HI}$

$$S = C_{HI}/A$$  32

TO DISPLAY BLOCK (34)

**FIG. 3**

TO BLOCK (36)

S  CURRENT SELECTIVITY OF CATALYST FROM BLOCK (28)

38

$$S > Z$$  YES

NO  (42)

36

39

**FIG. 4**

FORM MESSAGE

(40)

FIG. 5

EP 0 124 334 B1

## FIG. 6

```
                    CONTROL                    MESSAGE        TO BLOCK (42)
                    EXECUTIVE
                    CAT. STATUS
                    ACCOUNTING

                      56
    INPUT BY          NEW          NO           WAIT
    OPERATOR       CATALYST?

                      YES

                    INITIALIZE                 RECEIVE        MESSAGE
                    TIME = 0.0                                FROM (46)
                    TOTAL = 0.0
                    N REGEN = 0

    FROM            RECEIVE                     COMPUTE
    BLOCK (36)                                  TOTAL
                                                = TOTAL +
                                                TIME
                                                NREGEN =
                                                NREGEN + I

                    COMPUTE                     NREGEN         MESSAGE
                    TIME = TIME                 ≥ SREGEN       M# TO
                       + ΔT                                    DISPLAY
                                                               AND BLOCK
                                                NO             YES

                    TIME ≥      YES
                    STIME

                      NO
```